# EUROPEAN PATENT APPLICATION

(11) **EP 3 401 916 A1**
(43) Date of publication of application: **14.11.2018**
(21) Application number: 18171810.7
(22) Date of filing: 11.05.2018
(51) Int. Cl.: G16H 30/20, G16H 10/60, G16H 50/20, G16H 80/00

(54) **SYSTEM AND METHOD FOR VIRTUAL ENABLEMENT OF HEALTHCARE SERVICES**

(30) Priority: 11.05.2017 IN 201721016518
(71) Applicant: Tata Consultancy Services Limited, Maharashtra (IN)
(72) Inventor: KRISHNAMURTHY, Girish, 560066 Bangalore Karnataka (IN); GOEL, Rupesh, Patlipada, Thane West - 400 607 Maharashtra (IN); SESHADRI, Srikrishna, 560066 Bangalore Karnataka (IN)
(74) Representative: Goddar, Heinz J.

(57) **Abstract**

The disclosure presents a DiNC system and method to contextually communicate and share data amongst networked healthcare entities. It is configured to dynamically design virtual enablement services for the networked healthcare entities. DiNC is a service focused arrangement where communication is core to effect healthcare delivery. It allows user interaction both in context of a case management or in general communication. These interactions can be performed using multiple communication channels. It also facilitates healthcare services between the patients, who visit hospital and present in remote areas, with hospitals and doctors. The services provided are one amongst a catalog including but not limited to appointment scheduling, virtual doctor consultation, care follow ups of the patients and maintaining the case history of individual patients. It can predicts possible treatment outcome and possible epidemic outbreak. Further it provides triaging in healthcare provision by identifying level of care required and a probability of outcome.

## Description

### PRIORITY

The present application claims priority from Indian patent application number (201721016518), filed on May 11, 2017 the complete disclosure of which, in its entirety is herein incorporated by reference.

### FIELD OF THE INVENTION

The disclosure herein generally relates to telemedicine and, more particularly to a system and method for virtual enablement of healthcare services using telecommunication and information technology.

### BACKGROUND

As the population of a country grows and ages, the need for doctors and health care increases. This increased demand increases the need for medical professionals and specialized doctors in particular, within a short span of time. Additionally there is a complexity in delivering health care due to multi-fold patient diversity, inadequate infrastructure, asymmetry of information and ever increasing need for coordination and data driven decision making. The socio economic status, language, health awareness and social issues also create new complexities for all strata of society to access quality healthcare in a timely manner. Thus, there is an increased critical requirement for alternative means of obtaining and enabling quality health care that do not take too much time and can ensure quality healthcare services enablement using proper medical procedures and techniques.

Although it is still advantageous to contact medical professionals around the clock during emergencies, the healthcare industry realized that valuable time is wasted in commuting to and fro from hospital facilities for both patient and doctors. Time efficiency has also been a concern due to the time healthcare staff spent contacting the doctor, obtaining patient histories, lab results, x-ray images, or pharmaceutical information. This specialized time could have been better spent on patient care.

### SUMMARY OF THE INVENTION

Embodiments of the present disclosure present technological improvements as solutions to one or more of the above-mentioned technical problems recognized by the inventors in conventional systems.

In one embodiment, a computer implemented method to contextually communicate and share data amongst networked healthcare entities is provided. Further, the method to dynamically design virtually enablement services for the networked healthcare entities is also provided. The method comprising one or more steps such as providing a multi-modal interface to one or more users for healthcare communication by entering either text data or attaching an image or voice and seeking healthcare from any registered specialist, wherein each healthcare communication is tagged using context of communication, standard medical codes and procedure codes, receiving one or more queries of one or more users, wherein profiling each user based on behavior of communication of the one or more users, further wherein a patient is identified amongst the one or more users based the query received and the profile of the user, uploading a patient health record of the identified patient to create a central source of longitudinal patient health record, wherein the patient health record comprises both structured and unstructured data comprising medical images, scanned documents, text messages, medical records, voice and videos of the patient, identifying a healthcare delivery needs of the patient using an intelligent care triaging module and communicating the requirement of a medication attention and a treatment. Further herein, analyzing each event of the identified patient journey alongside the patient health record and mapping each event with the central source of patient health record, wherein the analysis points out anomalies in the patient healthcare events across time.

In another embodiment, a Digital Nerve Center (hereinafter referred as DiNC) system is configured to contextually communicate and share data amongst networked healthcare entities. Further the DiNC system is configured to dynamically design virtual enablement services for the networked healthcare entities. The DiNC system comprises a memory storing a plurality of instructions, one or more hardware processors communicatively coupled with the memory. The one or more hardware processors are configured by the plurality of instructions to execute one or more modules comprising a virtual communication module configured to provide a multi-modal interface to one or more users for healthcare communication by entering either text data, or attaching an image or voice and seeking a healthcare from any registered specialist, wherein the virtual communication module is further configured to tag each healthcare communication using context of the communication standard medical codes and procedure codes, a patient information management module configured to receive one or more queries of one or more users, wherein the patient information management module is further configured to identify a patient amongst the one or more users, further the patient information management module configured to profile the one or more users based on behavior of the communication of each user, a data consolidation module configured to upload the patient health record of the identified patient to create a central source of longitudinal patient health record, wherein the patient health record comprises of medical images, scanned documents, text messages, medical records, voice and videos of the patient, an intelligent care triaging module configured to identify healthcare delivery needs of the patient by using a communication channel to connect with the patient, wherein the communications are directed towards the patient requirement of medication attention and a treatment. Further herein, the DiNC system comprises an event analytics module configured to analyze each event of the patient journey alongside corresponding patient health record to map each event with the central source of patient health record, wherein the analysis points out anomalies in the patient healthcare events across time. Furthermore, the DiNC system comprising a broadcasting management module configured to identify each patient's preferred communication language and using the identified communication language to send customized messages based on the patient profiling.

It is to be understood that both the foregoing general description and the following detailed description are exemplary and explanatory only and are not restrictive of the invention, as claimed.

### BRIEF DESCRIPTION OF DRAWINGS

FIG. 1 illustrates a DiNC system for virtual enablement of healthcare services, according to an embodiment of a present subject matter;
FIG. 2(A) & 2(B) depict an overview of the DiNC system virtually enabling the coordination of the patients with any registered specialist, according to an embodiment of the present subject matter;
FIG.3(A) & 3(B) illustrate a flowchart explaining the detailed execution of an example service (appointment service) provided by the DiNC system, according to an embodiment of the present subject matter;
FIG. 4 illustrates the DiNC system that allows dynamic bundling of micro-services to facilitate business services, according to an embodiment of the present subject matter; and
FIG. 5 is a flowchart to illustrate a virtual enablement of healthcare services, according to an embodiment of the present subject matter.

### DETAILED DESCRIPTION OF THE INVENTION

The embodiments herein and the various features and advantageous details thereof are explained with reference to the non-limiting embodiments that are illustrated in the accompanying drawings and detailed in the following description. The examples used herein are intended merely to facilitate an understanding of ways in which the embodiments herein may be practiced and to further enable those of skill in the art to practice the embodiments herein. Accordingly, the examples should not be construed as limiting the scope of the embodiments herein.

The present disclosure provides herein a DiNC system and a method for virtual enablement of health care services to the patients visiting the hospital and present in remote areas. The disclosure connects the stakeholders i.e., patients, hospitals and doctors and provides services like appointment scheduling , virtual doctor consultation for patients present in remote location and maintaining the case histories of the patient in the structured format for future reference.

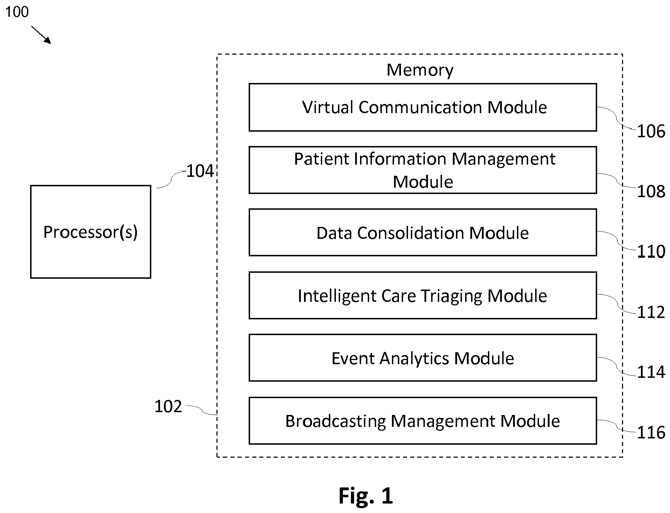

Referring FIG. 1 illustrating a DiNC system (100) to contextually communicate and share data amongst healthcare entities. The DiNC is a service focused system where communication is core to effect healthcare delivery. However, communication within the said arrangement happens through multiple channels across multiple stakeholders such as patient, doctors, specialists etc.

As shown in FIG. 1, the DiNC system (100) includes a memory (102), one or more hardware processor(s) (104) communicatively coupled with the memory (102), wherein the one or more hardware processors (104) are configured by the plurality of instructions to execute one or more modules. The one or more modules comprising a virtual communication module (106), a patient information management module (108), a data consolidation module (110), an intelligent care triaging module (112), an event analytics module (114) and a broadcasting management module (116). Although FIG. 1 shows example components of the DiNC system (100), in other implementations, the DiNC system (100) may contain fewer components, additional components, different components, or differently arranged components than depicted in FIG. 1.

The one or more hardware processor(s) (100) may include circuitry implementing, among others, audio and logic functions associated with the communication. The one or more hardware processor(s) (100) may include, among other things, a clock, an arithmetic logic unit (ALU) and logic gates configured to support operation of the processor(s) (100). The one or more hardware processor(s) (100) can be a single processing unit or a number of units, all of which include multiple computing units. The one or more hardware processor(s) (100) may be implemented as one or more microprocessors, microcomputers, microcontrollers, digital signal processors, central processing units, state machines, logic circuitries, and/or any devices that manipulate signals based on operational instructions. Among other capabilities, the one or more hardware processor(s) (100) is configured to fetch and execute computer-readable instructions and data stored in the memory (100).

The functions of the various elements shown in the figure, including any functional blocks labeled as "processor(s)", may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non-volatile storage. Other hardware, conventional, and/or custom, may also be included.

The memory (100) may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes. The memory (100), may store any number of pieces of information, and data, used by the DiNC system (100) to implement the functions of the DiNC system (100). The memory (100) may be configured to store information, data, applications, instructions or the like for enabling the DiNC system (100) to carry out various functions in accordance with various example embodiments. Additionally or alternatively, the memory (100) may be configured to store instructions which when executed by the processor(s) (100) causes the DiNC system (100) to behave in a manner as described in various embodiments. The one or more modules includes routines, programs, objects, components, data structures, etc., which perform particular tasks or implement particular abstract data types. The memory may include programs or coded instructions that supplement applications and functions of the DiNC system (100).

In the preferred embodiment of the disclosure, the virtual communication module (100) is configured for providing a collaboration and communication platform between different stakeholders of the DiNC system (100). The virtual communication module (100) is a multi-modal interface application that is inbuilt with virtual care workflow wherein the registered specialists are communicating with the patients using the DiNC system (100) through a chat interface wherein relevant information is present in the chat conversations. The multi-modal interface application also includes medical administrator who assists the registered specialists in chat conversation with the patient. It is to be noted that the multi-modal interface of the virtual communication module (106) can be used by one or more users for healthcare communication by entering either text data, or attaching an image or voice and seeking healthcare from any registered specialist. Further that the virtual communication module (106) is also configured to tag each healthcare communication using context of communication, standard medical codes and procedure codes.

The virtual communication module (106) is a cloud based clinical platform involving clinical data, virtual care and messaging services to enable seamless care coordination. The virtual communication module (106) connects patients with registered specialists in a virtual environment, exchanging data and enabling audio-video communication in a real time, secure and confidential manner through a clinical communicator.

Additionally, it would be appreciated that the DiNC system provides an enhanced contextual chat feature for real time communication (instant message and push notifications) in an online environment through the virtual communication module. In one aspect, wherein a communicator allows instant file sharing including audio and video files. The communicator leverages highly scaled web socket layer to facilitate real time data exchange and bidirectional tree based structure in chat conversations. The feature also works instantly across multiple platforms such as mobile phones, tablets and desktops.

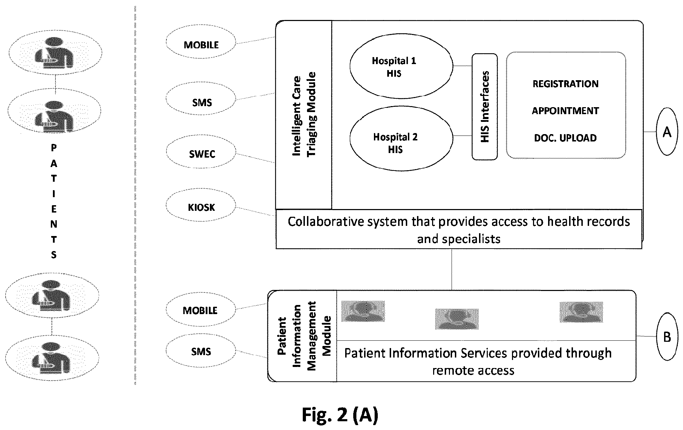

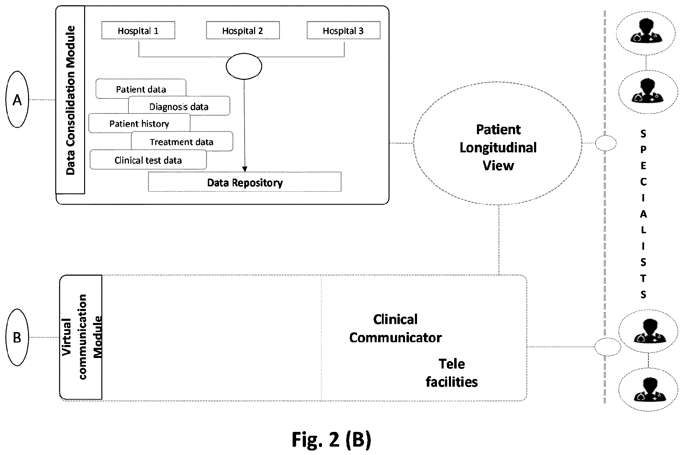

Referring FIG. 2 provides an overview of the DiNC system virtually enabling the coordination of the patients with the registered specialists. The chat based arrangement of the DiNC system (100) captures unstructured/informal communication (text, voice, graphics, and video) between the two parties such as a patient and a healthcare practitioner at a rural healthcare center and converts into a formal structured patient health record. Unstructured interaction between a patient and the healthcare practitioner is converted into the structured medical information using DiNC system (100).

The information can be marked as structured information using 'tags' in the unstructured text. The tags play a vital role to link in converting structured and unstructured data. The different steps involved in structuring the information is intelligent tagging, language conversion, algorithm (or technique(s)) to convert unstructured to structure patient health records, assist in context based search and integrate with local medical devices for instant monitoring of health parameters.

In the preferred embodiment of the disclosure, the patient information management module (108) is configured for handling one or more users' queries. Further herein, the one or more users' queries are provided with automated responses through SMS, mobile application and call channels. The automated responses are maintained as catalogue book of knowledge based on the user behavior, call response and clinical protocols.

Furthermore, the knowledge that is generated is used by the DiNC system (100) and is published through patient portal or application. Further herein, the patient information management module (108) is configured to identify a patient amongst the one or more users and to create a profile of each user based on behavior of communication of the corresponding user. The DiNC system (100) extracts metadata of received communication and records them under a patient communication index. In addition to this, the patient information management module (108) is also used as to prioritize one or more channels of communication and continuously improve the confidence of each communication channel.

Further, the patient information management module (108) maintains a middle layer of DiNC system (100) for personnel working in an integrated manner to manage the patient communication through inbound/outbound calls, SMS, emails etc. The patient information management module (108) also enables services such as call reminders, patient query management through toll free number and mass/bulk information services. Automated workflows are also setup so that tickets are routed to correct concerned information technology support teams for resolution.

In the preferred embodiment of the disclosure, the data consolidation module (110) is configured for uploading and archiving the patient health record of the identified patient to create a central source of longitudinal patient health record. The patient health record comprises of medical images, scanned documents, text messages, medical records, voice and videos of the patient. It is to be noted that the archived patient health record is stored categorically against each identified patient. In addition to this, the storing the structured data alongside the unstructured data elements can create a contextual summary for care enablement and a service design decision.

The data consolidation module (110) consolidates large scale structured and unstructured data from other modules of the DiNC system (100) into a repository to build a single source for all patient data. The data consolidation module (110) extracts metadata so that it can be presented as meaningful analytical visuals for clinical consumption. The repository populates the data from other modules on a daily basis and the patient health record is improved as encounters over a period of time. Rendition of this data enables a medical practitioner to gain access to contextual data of a patient while rendering any of the DiNC services named a few as medical queries, appointment triaging, patient preparation, active patient management, counselling and analytical services.

In the preferred embodiment of the disclosure, the intelligent care triaging module (112) is configured to identify at least one healthcare delivery need of the identified patient by using a communication channel of one or more channels of communication to connect/interface with the one or more users. The healthcare delivery needs are directed towards the patient requirement of medication attention and treatment. It is an arrangement to triage medical needs of the patient using standardized clinical protocols, document analysis and real-time communication with specialists. Further, the intelligent care triaging module (112) is also configured to analyze the medication attention to assist the identified patient to visit either as a physical presence or in a digital presence using a communication channel interface managed by a middle layer of clinical service personnel.

The intelligent care triaging module (112) is a collaborative resource for scheduling and notification. It maintains real time centralized appointment calendars for hospital resources such as the medical practitioners. The intelligent care triaging module (112) maintains separate calendars for virtual consultations with registered specialists. It allows registration and appointment services such as booking, cancellation and rescheduling based on one or more requests received through multiple channels such as kiosks, mobile phone, SMS etc. as shown in FIG. 2. Further, it also allows integration with any hospital HIS (Hospital Information System).

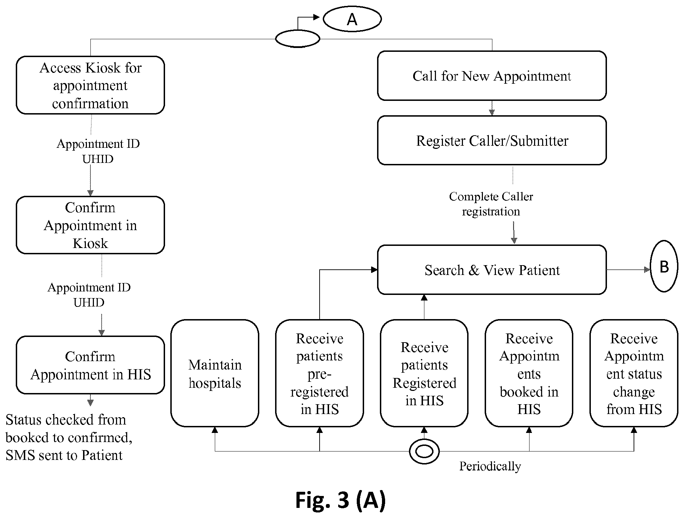

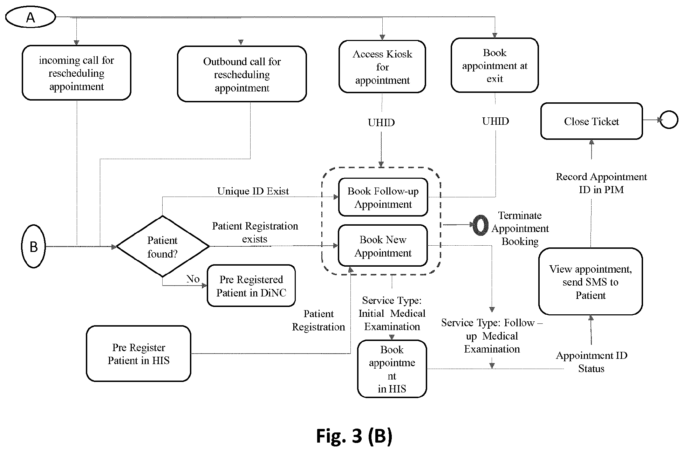

Referring FIG. 3 which is a flowchart explaining the detailed execution of an example service (appointment service) provided by the DiNC system (100). The services are provided to the patients who visit the hospital and the patients that are located in remote areas. Herein, the DiNC system (100) is configured to facilitate centralized appointment optimizing DiNC system (100) by identifying the availability of critical resources like specialists for virtual consultation and facilitate appointment confirmation. The DiNC system (100) utilizes instructions that matches the needs of the patient vis-à-vis a specialist available in the nearest vicinity. The DiNC system (100) can book appointments for patients, including in-person visit as well as virtual consultations. This includes omni-channel access to appointment booking (phone, kiosk, single window exit counters), cancellation, rescheduling, bulk cancellations/transfers, appointment reminders, dropout tracking and other operational activities related to appointment. A new appointment is an appointment booked for the first time patients to the hospital. The patient information is not available in hospital and so the patient will need to be pre-registered prior to granting an appointment. In such cases, site triaging, patient category determination may also need to be done by nurses/doctors in a DiNC arrangement. A follow-up appointment is an appointment for follow-up patients to the hospital. The patient is registered at the hospital and the hospital specific Unique Health Information Identifier (UHID) is known or searchable for the patient. In such cases, no triaging is expected and appointment can be booked for the UHID. It would be appreciated that in appointment reminders, a dedicated person of the DiNC arrangement proactively calls the patients and reminds them of upcoming appointments. This is intended to improve adherence to pre-booked appointment. Even the dedicated person of the DiNC arrangement can cancel appointments due to unavailability of the medical practitioners, an equipment in the hospital or unavailability of the patient itself. Appointments can also be rescheduled to a later time when the medical practitioner and/or patient are available.

In addition to this, the DiNC system (100) is also configured for dynamically selecting at least one or more micro-services to define a use case depending upon the business need. The DiNC system (100) further allows development of a workflow to orchestrate the interconnection and execution of the selected one or more micro-services to fulfill a request from the one or more users.

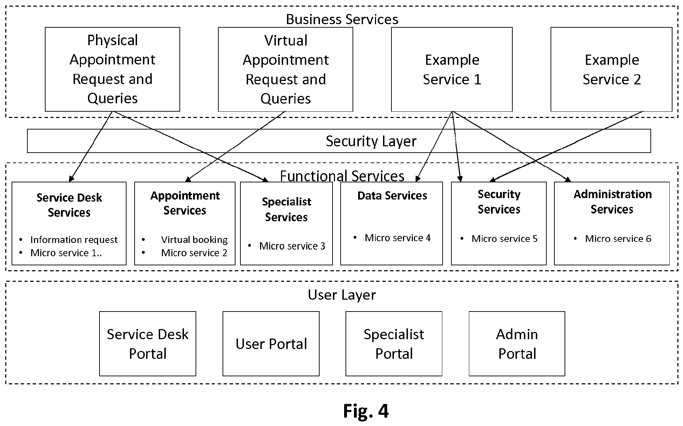

Referring FIG. 4 illustrating one or more features of the DiNC system (100) that allows dynamic bundling of micro-services to facilitate business services. The DiNC system (100) comprising a micro-service platform for developing and executing a plurality of micro-services. Each micro-service can be configured and deployed independently to exclude one or more functions. These micro-services are then clustered together in a plurality of ways to create new workflows and business services. The micro-services are designed to enable convenient, faster and quality healthcare delivery, including but not limited to, registration, appointment booking, virtual consultation, counselling, discharge coordination etc. The DiNC system (100) further allows development of a workflow to orchestrate the interconnection and execution of the selected one or more micro-services to fulfill a request.

In the preferred embodiment of the disclosure, an event analytics module (114) which is configured to analyze each event of the patient journey alongside corresponding patient health record and to map each event with the central source of longitudinal patient health record. The analysis may point out anomalies in the patient healthcare events across time. Further, based on data captured across multiple sources, the DiNC system (100) uses advanced analytics to create use cases to predict possible patient outcomes for a treatment plan, a possible epidemic outbreak, comparison of cohorts etc. The use cases are primarily aimed towards facilitating advanced research to improve healthcare delivery. The prediction is based on recognizing the patterns of patients visiting doctor, correlative analytics to match clinical data and hospital visit, drug compliance and related comorbidity to enable communication and follow-up ability to predict probable events in a patient health journey from both structured and unstructured data and able to predict patient's future medical needs.

In another embodiment of the disclosure, the DiNC system (100) comprises a broadcasting management module (116). The broadcasting management module (116) is configured to identify a preferred communication language of the identified patient and using the identified communication language to send customized messages to the identified patient based on the patient profiling. Further, the profiling is also used to 'push' messages in multi-modal format various messages facilitating care delivery.

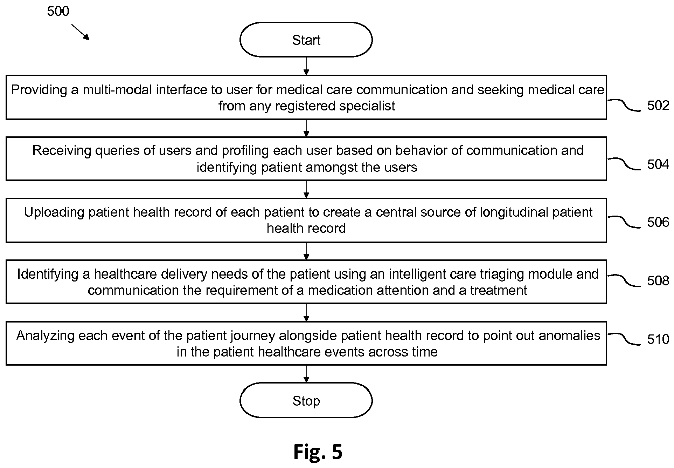

Referring FIG. 5 illustrating a computer implemented method (500) to contextually communicate and share data amongst healthcare entities using a digital nerve center DiNC system (100). The DiNC is a service focused system where communication is core to effect healthcare delivery. Further wherein the method (500) to dynamically design virtual enablement services for the networked healthcare entities. The DiNC system (100) is also configured to implement the method of dynamically selecting at least one or more micro-services to define a use case depending upon the business need.

Initially, at the first step (502), providing a multi-modal interface to one or more users for a healthcare communication by entering either text data or attaching an image or a voice and seeking healthcare from any registered specialist using a virtual communication module (106). It would be appreciated that each healthcare communication is tagged using context of communication, standard medical codes and procedure codes.

At the next step (504), one or more queries of the one or more users are received at a patient information management module (108) of the DiNC system (100). The one or more users' queries are provided with automated responses through SMS, mobile application and call channels. The automated responses are maintained as catalogue book of knowledge based on the user behavior, call response and clinical protocols. The knowledge that is generated is used by the DiNC system (100) and is published through patient portal or application.

It is to be noted that the each user registering with the DiNC system (100) is by nature not classified as a patient. There are certain validation data points to identify a patient amongst the one or more users registered with the DiNC system (100). There are also criteria to further classify the identified patient into an active patient and a passive patient, for example, if someone is potentially going to visit any healthcare facility in the next 12 months, he/she would be classified as an active patient. Further, it allows indexing an active patient based on the disease/presenting illness to auto generate management protocol and its corresponding disease category.

At the next step (506), a patient health record of the identified patient is uploaded at a data consolidation module (110) to create a central source of longitudinal patient health record. The patient health record comprises of medical images, scanned documents, text messages, medical records, voice and videos of the patient. The said patient health record is used by DiNC service providers to identify each patient's healthcare delivery needs. It is also an arrangement to nominalize, normalize and de-dupe patient records. Further, the method (500) comprises archiving the patient health record categorically at the data consolidation module (110), wherein archiving of structured data alongside the unstructured data elements of the patient health record is done to create a contextual summary for the healthcare delivery needs.

At the step (508), wherein at least one healthcare delivery need of the identified patient to be identified using an intelligent care triaging module (112) and communicating the requirement of medication attention and treatment by using a communication channel of one or more channels of communication to connect/interface with the one or more users. Triaging in healthcare provision identifies the level of care required and outcome probability such that critical resources can be deployed for maximizing positive outcomes from care. The one or more channels of communication can be used to connect with the identified patient or classified 'active patient' and these communication are directed towards their requirement of medical attention and treatment. The medical attention thus identified are further analyzed by the DiNC system (100) for visit of the identified patient as physical presence or as in a digital presence using the communication interface managed by clinical service personnel. If the medical attention is in digital presence, the clinical service personnel within the DiNC system (100) may assist to connect the identified patient and the registered specialist using video and audio technology. The medical attention in physical presence may be further processed with two criteria as in fastest available care and nearest to the place of geographic residence of the patient.

At the final step (510), analyzing each event of the identified patient journey alongside corresponding patient health record of the identified patient and mapping each event with the central source of longitudinal patient health record to point out anomalies in the patient healthcare events across time. Furthermore, the analysis also points out key clinical alerts which comprises non-communicable diseases like diabetes, cancer, heart problem and communicable disease like tuberculosis, HIV etc. It is to be noted that the key clinical alerts are pre-configured in the DiNC system (100) and it also allows the DiNC system (100) to bring meaningfulness to capturing unstructured data into the DiNC system (100).

In another embodiment of the disclosure, wherein the method (500) comprising identifying a preferred communication language of the identified patient using a broadcasting management module (116) of the DiNC system (100) to send customized messages to the identified patient based on the profile of the identified patient. It would be appreciated that the population health is significantly based on constant, relevant and relative communication and messaging. It is to be noted that the DiNC system (100) is profiling patients based on their medical condition, demographics, and preference etc. to push messages in multi-modal format facilitating healthcare delivery. Further to the identification of the preferred communication language, the DiNC system (100) is also having a messaging repository wherein rules are set to send custom messages based on the patient information management module (108) profiling. Furthermore, the DiNC system (100) is also used to generate and auto forward messages at a point in conversation where the user no longer finds the auto responses valid for further follow-up by the clinical service personnel.

The order in which the method(s) and system(s) are described is not intended to be construed as a limitation, and any number of the described method blocks can be combined in any order to implement the method or system, or an alternative method or system. Additionally, individual blocks may be deleted from the methods without departing from the spirit and scope of the subject matter described herein. Furthermore, the method and system can be implemented in any suitable hardware, software, firmware, or combination thereof.

In an implementation, one or more of the method(s) or system(s) described herein may be implemented at least in part as instructions embodied in a non-transitory computer-readable medium and executable by one or more computing devices. In general, a processor (for example a microprocessor) receives instructions, from a non-transitory computer-readable medium, for example, a memory, and executes those instructions, thereby performing one or more method(s), including one or more of the method(s) described herein. Such instructions may be stored and/or transmitted using any of a variety of known computer-readable media. The method can be implemented on computer, smart phones, tablets, kiosks and any other similar device.

The preceding description has been presented with reference to various embodiments. Persons having ordinary skill in the art and technology to which this application pertains appreciate that alterations and changes in the described structures and methods of operation can be practiced without meaningfully departing from the principle, spirit and scope.

## Claims

1. A digital nerve center (DiNC) system (100) comprising:
a memory (102) storing a plurality of instructions;
one or more hardware processors (104) communicatively coupled with the memory (102), wherein the one or more hardware processors (104) are configured by the plurality of instructions to execute modules comprising:
a virtual communication module (106) configured to provide a multi-modal interface to one or more users for a healthcare communication either by entering text data, or attaching an image or voice and seeking healthcare from any registered specialist, wherein the virtual communication module (106) is further configured to tag each healthcare communication using context of the communication, standard medical codes and procedure codes;
a patient information management module (108) configured to receive one or more queries of one or more users and to identify a patient amongst the one or more users, further the patient information management module (108) configured to create a profile of each user based on behavior of communication of each user;
a data consolidation module (110) configured to create a central source of longitudinal patient health record, wherein the patient health record comprises of medical images, scanned documents, text messages, medical records, voice and videos of the patient, further wherein the data consolidation module is configured to store structured data alongside unstructured data in the central source of longitudinal patient health record to create a contextual summary for healthcare delivery needs;
an intelligent care triaging module (112) configured to connect the registered specialist with the identified patient for the healthcare delivery needs of the identified patient using a communication channel of one or more communication channels, wherein the healthcare delivery needs are directed towards the patient requirement of medication attention and treatment; and
an event analytics module (114) configured to analyze each event of the identified patient journey alongside a corresponding patient health record and to map each event with the central source of longitudinal patient health record, wherein the analysis points out anomalies in the identified patient healthcare events across time.

2. The DiNC system (100) of claim 1, further comprising:
a broadcasting management module (116) configured to identify a preferred communication language of the identified patient and using the identified preferred communication language to send a customized message based on the created profile of the identified patient.

3. The DiNC system (100) of claim 1, wherein the patient information management module (108) is further configured to extract metadata of a received communication and record under a patient communication index.

4. The DiNC system (100) of claim 1, wherein the patient information management module (108) is further configured to prioritize one or more channels of communication and for continuously improving the confidence of each communication channel.

5. The DiNC system (100) of claim 1, wherein the intelligent care triaging module (112) is further configured to analyze medication attention to assist the identified patient to visit either as a physical presence or in a digital presence using a communication channel interface managed by a middle layer of a clinical service personnel.

6. A computer implemented method (500) comprising:
providing a multi-modal interface to one or more users for healthcare communication by entering either text data, or attaching an image or a voice and seeking healthcare from any registered specialist using a virtual communication module (106), wherein each healthcare communication is tagged using context of communication, standard medical codes and procedure codes;
receiving, at a patient information management module (108), one or more queries of the one or more users and creating profile of each user based on behavior of the communication of the corresponding user, wherein a patient is identified amongst the one or more users based the query received and the profile of the user;
uploading, at a data consolidation module (110), a patient health record of the identified patient to create a central source of longitudinal patient health record, wherein the patient health record comprises of medical images, scanned documents, text messages, medical records, voice and videos of the patient;
connecting the registered specialist with the identified patient using an intelligent care triaging module (112) and communicating the healthcare delivery needs of the identified patient using a communication channel or one or more communication channels, wherein the healthcare delivery needs are directed towards the identified patient requirement of a medication attention and a treatment; and
analyzing each event of the identified patient journey alongside a corresponding patient health record and mapping each event with the central source of longitudinal patient health record, wherein the analysis points out anomalies in the identified patient healthcare events across time.

7. The method (500) of claim 6, further comprising identifying a preferred communication language of the identified patient and using the identified preferred communication language to send a customized message based on the identified patient profiling.

8. The method (500) of claim 6, further comprising extracting metadata of the received communication between the identified patient and the registered specialist and recording the extracted metadata under a patient communication index.

9. The method (500) of claim 6, further comprising prioritizing one or more channels of communication and continuously improving the confidence of each communication channel.

10. The method (500) of claim 6, wherein storing structured data alongside unstructured data in the central source of longitudinal patient record to create a contextual summary for the healthcare delivery needs.

11. The method (500) of claim 6, further comprising analyzing the medication attention to assist the identified patient to visit either as physical presence or in a digital presence using a communication channel interface managed by a middle layer of clinical service personnel.

12. A non-transitory computer readable medium storing one or more instructions which when executed by a processor on a system cause the processor to perform a method comprising one or more steps of:
providing a multi-modal interface to one or more users for healthcare communication by entering either text data, or attaching an image or a voice and seeking healthcare from any registered specialist using a virtual communication module (106), wherein each healthcare communication is tagged using context of communication, standard medical codes and procedure codes;
receiving, at a patient information management module (108), one or more queries of the one or more users and creating profile of each user based on behavior of the communication of the corresponding user, wherein a patient is identified amongst the one or more users based the query received and the profile of the user;
uploading, at a data consolidation module (110), a patient health record of the identified patient to create a central source of longitudinal patient health record, wherein the patient health record comprises of medical images, scanned documents, text messages, medical records, voice and videos of the patient;
connecting the registered specialist with the identified patient using an intelligent care triaging module (112) and communicating the healthcare delivery needs of the identified patient using a communication channel or one or more communication channels, wherein the healthcare delivery needs are directed towards the identified patient requirement of a medication attention and a treatment; and
analyzing each event of the identified patient journey alongside a corresponding patient health record and mapping each event with the central source of longitudinal patient health record, wherein the analysis points out anomalies in the identified patient healthcare events across time.
